# EUROPEAN PATENT SPECIFICATION

(11) **EP 3 374 029 B1**
(45) Date of publication and mention of the grant of the patent: **20.07.2022**
(21) Application number: 16717207.1
(22) Date of filing: 15.03.2016
(51) Int. Cl.: A61K 33/06, A61P 41/00, A61P 11/00, A61P 35/00, C01B 39/14

(54) **A ZEOLITE USED IN PLEURODESIS**
ZEOLITH FÜR PLEURODESE
ZÉOLITHE UTILISÉE DANS UNE PLEURODÈSE

(30) Priority: 09.11.2015 TR 201513993
(43) Date of publication of application: 19.09.2018
(73) Proprietor: Yeditepe Universitesi, 34755 Istanbul (TR)
(72) Inventor: AYDIN, Ahmet, Istanbul (TR); CHAREHSAZ, Mohammed, 34755 Atasehir Istanbul (TR); SIPAHI, Hande, Istanbul (TR); DUMAN, Gulengul, Istanbul (TR); CELIK, Hayati, Atasehir/Istanbul (TR); BAC, Nurcan, Lubbock, TX 79409 (US); ERCAN, Sina, Atasehir/Istanbul (TR)
(74) Representative: Dericioglu, E. Korhan
(86) International application number: PCT/TR2016/050070
(87) International publication number: WO 2017/082840

(56) References cited:
- J. MAXWELL: "The Production of Pleural Adhesions by Kaolin Injection", THORAX, vol. 9, no. 1, 1 March 1954 (1954-03-01), pages 10-13, XP055274421, GB ISSN: 0040-6376, DOI: 10.1136/thx.9.1.10
- BAYIR A ET AL: "Comparison of the topical haemostatic efficacy of nano-micro particles of clinoptilolite and kaolin in a rat model of haemorrhagic injury", EUROPEAN JOURNAL OF TRAUMA AND EMERGENCY SURGERY, SPRINGER BERLIN HEIDELBERG, BERLIN/HEIDELBERG, vol. 42, no. 1, 14 March 2015 (2015-03-14) , pages 77-86, XP035624655, ISSN: 1863-9933, DOI: 10.1007/S00068-015-0506-Z [retrieved on 2015-03-14]
- LYNDA B. WILLIAMS ET AL: "Evaluation of the medicinal use of clay minerals as antibacterial agents", INTERNATIONAL GEOLOGY REVIEW, vol. 52, no. 7-8, 6 May 2010 (2010-05-06), pages 745-770, XP055274425, US ISSN: 0020-6814, DOI: 10.1080/00206811003679737
- ERSIN DEMIRER ET AL: "Clinical and Prognostic Features of Erionite-Induced Malignant Mesothelioma", YONSEI MEDICAL JOURNAL, vol. 56, no. 2, 1 January 2015 (2015-01-01), page 311, XP055274431, KI ISSN: 0513-5796, DOI: 10.3349/ymj.2015.56.2.311
- AYELE LIJALEM ET AL: "Conventional versus alkali fusion synthesis of zeolite A from low grade kaolin", APPLIED CLAY SCIENCE, ELSEVIER, AMSTERDAM, NL, vol. 132, 25 July 2016 (2016-07-25), pages 485-490, XP029761374, ISSN: 0169-1317, DOI: 10.1016/J.CLAY.2016.07.019
- TEKIN RUMEYSA ET AL: "Effect of reaction mixture composition and silica source on size distribution of zeolite X crystals", JOURNAL OF CRYSTAL GROWTH, ELSEVIER, AMSTERDAM, NL, vol. 411, 18 November 2014 (2014-11-18), pages 45-48, XP029122585, ISSN: 0022-0248, DOI: 10.1016/J.JCRYSGRO.2014.11.017
- Anonymous: "FAU Framework Type Data Faujasite", , 14 December 2018 (2018-12-14), XP055710516, Retrieved from the Internet: URL:https://www.epo.org/ [retrieved on 2020-07-01]
- WARZYWODA J ET AL: "Growth of zeolites A and X in low earth orbit", JOURNAL OF CRYSTAL GROWTH, ELSEVIER, AMSTERDAM, NL, vol. 220, no. 1-2, 15 November 2000 (2000-11-15), pages 140-149, XP004219442, ISSN: 0022-0248, DOI: 10.1016/S0022-0248(00)00659-X
- ANONYMOUS: "LTA - PM3M - FRAMEWORK TYPE DATA - TYPE MATERIAL: LINDE TYPE A", STRUCTURE COMMISSION OF THE INTERNATIONAL ZEOLITE ASSOCIATION (IZA-SC) , 26 August 2010 (2010-08-26), pages 194-195, XP002598626, Retrieved from the Internet: URL:http://izasc.ethz.ch/fmi/xsl/IZA-SC/At las_pdf/LTA.pdf [retrieved on 2010-08-26]
- Anonymous: "Crystallographic information file for Na-X Hydrated", , 14 December 2018 (2018-12-14), XP055710528, Retrieved from the Internet: URL:https://www.epo.org/ [retrieved on 2020-07-01]
- WARZYWODA J ET AL: "Synthesis of large zeolite X crystals", JOURNAL OF CRYSTAL GROWTH, ELSEVIER, AMSTERDAM, NL, vol. 204, no. 4, 1 August 1999 (1999-08-01), pages 539-541, XP004181438, ISSN: 0022-0248, DOI: 10.1016/S0022-0248(99)00235-3
- John A. Thomas ET AL: "Toxicological Assessment of Zeolites", JOURNAL OF THE AMERICAN COLLEGE OF TOXICOLOGY, vol. 11, no. 3, 1 May 1992 (1992-05-01), pages 259-273, XP055470460, US ISSN: 0730-0913, DOI: 10.3109/10915819209141860
- ZARKOVIC NEVEN ET AL: "Anticancer and antioxidative effects of micronized zeolite clinoptilolite", ANTICANCER RESEARCH, INTERNATIONAL INSTITUTE OF ANTICANCER RESEARCH, GR, vol. 23, no. 2B, 1 March 2003 (2003-03-01) , pages 1589-1596, XP009165602, ISSN: 0250-7005

## Description

### Technical Field

The present invention relates to a zeolite used in pleurodesis which is performed in case of liquid accumulation (pleural effusion) or air accumulation (pneumothorax) in the pleural cavity of lungs.

### Prior Art

Pneumothorax is the accumulation of air between the visceral and parietal pleurae surrounding the lungs. When an individual with an existing pulmonary disease develops pneumothorax, it is called the secondary spontaneous pneumothorax. Pleurodesis is the medical procedure in which the pleural cavity is obliterated through adhesion of parietal and visceral pleurae which is achieved by introducing a sclerosing agent into the pleural cavity through a chest tube or thoracoscope. Pleurodesis aims to prevent the recurrence of pleural effusion and pneumothorax, and achieve full expansion of the lung. When tube thoracostomy fails, pleurodesis can be performed safely with low recurrence rates. It is also often required in lung cancer cases.

Various pharmaceuticals are used for pleurodesis. Among these pharmaceuticals, sterile talc powder is the most widely used one. It was first used by a surgeon named Norman Bethune in 1935.1 The talc powder prepared sterilely and usually formulated to be 4 grams is administered to the pleura by a specialist in thoracic surgery. There are two types of administrations of talc powder. The first one is the introduction of 4 grams of sterile talc powder in suspension into the pleural cavity
¹ Janssen, JP., Collier, G., Astoul, P., Tassi, GF., Noppen, M., Panadero-Rodriguez, F., Loddenkemper, R., Herth, FJF., Gasparini S., Marquette, CH., Becke, B., Froudarakis, MF., Driesen, P., Bolliger, CT., Tschopp, JM.: Safety of Pleurodesis with talc poudrage in malignant pleural effusion: a prospective cohort study. Lancet, 369:1535-39, 2007 using a catheter while the second method involves spraying the talc powder in aerosol prepared in a pressure vessel into the pleura using a cannula.

J. MAXWELL: "The Production of Pleural Adhesions by Kaolin Injection", THORAX, vol. 9, no. 1, 1 March 1954 (1954-03-01), pages 10-13, XP055274421, discloses the use of a suspension of kaolin in pleurodesis procedures.

### Summary of the Invention

The present invention is directed to zeolite X for use as a chemical agent in pleurodesis procedures performed in the cases of pneumothorax and/or pleural effusion as a chemical agent. In another aspect, the present invention relates to zeolite X for use in the pleurodesis procedures performed in pleural effusions and/or pneumothorax secondary to lung cancer as in the form of hydrated aluminum silicate showing both a pleurodesis chemical agent and cytotoxic agent effect.

In an aspect of the present invention zeolite X is used in powdered form.

Yet another aim of the present invention is to make use of the cytotoxic effect of zeolites on cancer cells as defined in the appended claims.

### Detailed Description of the Invention

The present invention relates to zeolite X for use as a chemical agent in pleurodesis procedures performed in pneumothorax and/or pleural effusions. In order to realize the aim of the present invention, the synthetic zeolite X is used.

Due to its cytotoxic effect, the zeolite of the invention also kills cancer cells in the site of administration in lung cancer cases. Thus, it has a dual effect as both a pleurodesis chemical agent and cytotoxic agent in the treatment of pleural effusions and/or pneumothorax secondary to lung cancer, as well as in the treatment of lung cancer itself. The chemical composition of the relevant zeolite, which is of mineral origin, is hydrated aluminum silicate.

The preferred embodiment of the invention is to introduce the powdered zeolite suspension into the pleural cavity through a catheter. Most preferably, the diameter of a powdered zeolite particle is approximately 20 micrometers.

In another embodiment of the invention, the powdered zeolite material is sprayed into the pleural cavity through aerosol tubes during pleurodesis.

## Claims

1. Zeolite X for use as a chemical agent in pleurodesis procedures performed in the cases of pneumothorax and/or pleural effusion as a chemical agent.

2. Zeolite X for use in the pleurodesis procedures performed in pleural effusions and/or pneumothorax secondary to lung cancer as in the form of hydrated aluminum silicate showing both a pleurodesis chemical agent and cytotoxic agent effect.

3. Zeolite X in suspension form for use in pleurodesis procedures according to Claim 1 or 2, introduced into the pleural cavity using a catheter.

4. Zeolite X in powdered form for use in pleurodesis procedures according to Claim 1 or 2, introduced into the pleural cavity using aerosol tubes.

## Patentansprüche

1. Zeolith X zur Verwendung als ein chemisches Mittel bei Pleurodeseverfahren, die bei Pneumothorax und/oder Pleuraerguss als ein chemisches Mittel durchgeführt werden.

2. Zeolith X zur Verwendung in Pleurodeseverfahren bei Pleuraergüssen und/oder Pneumothorax infolge von Lungenkrebs als Aluminiumsilikathydrat, das sowohl eine Wirkung als chemisches Pleurodesemittel als auch als zytotoxisches Mittel zeigt.

3. Zeolith X in Form einer Suspension zur Verwendung bei Pleurodeseverfahren nach Anspruch 1 oder 2, die unter Verwendung eines Katheters in die Pleurahöhle eingebracht wird.

4. Zeolith X in Form eines Pulvers zur Verwendung bei Pleurodeseverfahren nach Anspruch 1 oder 2, das unter Verwendung der Aerosolschläuche in die Pleurahöhle eingebracht wird.

## Revendications

1. Zéolite X à utiliser comme un agent chimique dans les procédures de pleurodèse effectuées dans les cas de pneumothorax et/ou épanchement pleural comme un agent chimique.

2. Zéolite X à utiliser dans les procédures de pleurodèse effectuées dans les épanchements pleuraux et/ou pneumothorax secondaires au cancer du poumon comme sous la forme de silicate d'aluminium hydraté montrant à la fois un effet d'agent chimique de pleurodèse et d'agent cytotoxique.

3. Zéolite X sous la forme de suspension à utiliser dans les procédures de pleurodèse selon la revendication 1 ou 2, introduite dans la cavité pleurale en utilisant un cathéter.

4. Zéolite X sous la forme de poudre à utiliser dans les procédures de pleurodèse selon la revendication 1 ou 2, introduite dans la cavité pleurale en utilisant les tubes d'aérosol.
